# EUROPEAN PATENT APPLICATION

(11) **EP 1 925 252 A1**
(43) Date of publication of application: **28.05.2008**
(21) Application number: 06797083.0
(22) Date of filing: 30.08.2006
(51) Int. Cl.: A61B 1/00

(54) **TIP STRUCTURE PART OF ENDOSCOPE**

(30) Priority: 14.09.2005 JP 2005267188
(71) Applicant: Olympus Corporation, Tokyo 151-0072 (JP)
(72) Inventor: TAKEUCHI, Akio ;c/o Intell. Property Support Dep., Hachioji-shi, Tokyo 192-8512 (JP)
(74) Representative: Winter, Brandl, Fürniss, Hübner Röss, Kaiser, Polte Partnerschaft Patent- und Rechtsanwaltskanzlei
(86) International application number: PCT/JP2006/317110
(87) International publication number: WO 2007/032210

(57) **Abstract**

An endoscope whose distal end forming portion can be readily manufactured and whose manufacturing cost is reduced is provided.

This endoscope includes a hollow distal end member (24) provided at a distal end of an endoscope insertion portion (14), a built-in component accommodating portion (25) that is formed of an inside of the distal end member (24) and accommodates a distal end of an elongated built-in component (26, 27) which is inserted through the insertion portion (14), a built-in component holding portion (42a, 42b, 42c, 42d) that holds a distal end of the built-in component (26, 27) to be positioned with respect to the distal end member (24), a filler accommodating portion (44) that is provided in the distal end member (24), a first and a second communicating portions (46a and 46b) that allow the outside of the distal end member (24) to communicate with the filler accommodating portion (44), and a filler that is accommodated in the filler accommodating portion (44) from the first communicating portion (46a) with the second communicating portion (46b) being used as an air vent, and covers at least a part of the built-in component (26, 27).

## Description

### Technical Field

The present invention relates to an endoscope including a distal end forming portion constituting a distal end thereof.

### Background Art

An endoscope is conventionally used whose insertion portion is inserted into a body cavity to perform observation of the inside of the body cavity and others. Various kinds of built-in components such as an observation optical system, an illumination optical system and others are inserted through the insertion portion of the endoscope. Distal ends of these built-in components are accommodated and fixed in a distal end member of a distal end forming portion arranged at the distal end of the insertion portion. That is, a through hole is formed in the distal end member in accordance with each built-in component, and the distal end of each built-in component is inserted and fitted in this through hole. Further, an adhesive, a heat insulating material or the like is put between an inner peripheral surface of the through hole and an outer peripheral surface of the built-in component to wrap the built-in component in order to fix the built-in component in the distal end member and protect, thermally insulate, and liquid-tightly hold the built-in component (see, e.g., Jpn. Pat. Appln. KOKAI Publication No. 42201-1999).

### Disclosure of Invention

Various kinds of built-in components must be accurately positioned in the distal end forming portion. For example, when a structure in which a distal end of an image guide is connected with an object lens group at a distal end is used as an observation optical system, the object lens group and the distal end of the image guide must be accurately positioned to obtain a sufficiently clear observation image. Therefore, the distal end member that is small must be accurately machined into a complicated shape. Furthermore, a dedicated jig is required to fill the small distal end member including a complicated shape with an adhesive and the like, and an operation of wiping off an adhesive and the like becomes troublesome since the adhesive and the like overflows along the complicated shape. As explained above, manufacture of the distal end forming portion of the endoscope is a bother, and a manufacturing cost of the endoscope is increased.

The present invention has been developed in view of the above problem, and it is an object of the present invention to provide an endoscope whose distal end forming portion can be readily manufactured and whose manufacturing cost is reduced.

In an aspect of the present invention, an endoscope distal end forming portion is characterized by comprising a hollow distal end member provided at a distal end of an elongated endoscope insertion portion that is inserted into a body cavity; a built-in component accommodating portion that is formed of an inside of the distal end member, and accommodates a distal end of an elongated built-in component which is inserted through the insertion portion; a built-in component holding portion that holds the distal end of the built-in component to be positioned with respect to the distal end member; a filler accommodating portion that is provided inside of the distal end member; a first and a second communicating portions that allow the outside of the distal end member to communicate with the filler accommodating portion; and a filler that is accommodated in the filler accommodating portion from the first communicating portion with the second communicating portion being used as an air vent, and covers at least a part of the built-in component.

In a preferred aspect of the present invention, the endoscope distal end forming portion is characterized in that the filler accommodating portion is formed of a part of the built-in component accommodating portion except a part occupied by the built-in component.

In a preferred aspect of the present invention, the endoscope distal end forming portion is characterized in that the distal end member includes a substantially cylindrical shape including both end walls and a circumferential wall, one of the first and the second communicating portions is formed to pass through one end wall, and the other of the first and the second communicating portions is formed to pass through the circumferential wall.

In a preferred aspect of the present invention, the endoscope distal end forming portion is characterized in that the distal end member includes a substantially cylindrical shape including both end walls and a circumferential wall, and the second communicating portion is formed to pass through one end wall.

In a preferred aspect of the present invention, the endoscope distal end forming portion is characterized in that the distal end member includes a substantially cylindrical shape including both end walls and a circumferential wall, and the first and the second communicating portions are formed to pass through one end wall.

In a preferred aspect of the present invention, the endoscope distal end forming portion is characterized in that the distal end member includes a substantially cylindrical shape including a front end wall, a rear end wall, and a circumferential wall, and the second communicating portion is formed to pass through the rear end wall.

In a preferred aspect of the present invention, the endoscope distal end forming portion is characterized in that the first and the second communicating portions are formed to pass through the distal end member in such a manner that the first and the second communicating portions are continuous with each other.

In a preferred aspect of the present invention, the endoscope distal end forming portion is characterized in that the endoscope distal end forming portion further comprises a hollow filler accommodating member that is provided in the distal end member and includes the filler accommodating portion formed therein.

In a preferred aspect of the present invention, the endoscope distal end forming portion is characterized in that the built-in component includes an observation optical system.

In another aspect of the present invention, an endoscope is characterized by comprising the above endoscope distal end forming portion.

According to the present invention the distal end forming portion can be easily manufactured, and a manufacturing cost of the endoscope is reduced.

### Brief Description of the Drawings

FIG. 1 is a perspective view showing an endoscope according to a first embodiment of the present invention;
FIG. 2A is a schematic perspective view showing a distal end forming portion of the endoscope according to the first embodiment of the present invention in a state before filling a filler;
FIG. 2B is a longitudinal cross-sectional view showing the distal end forming portion of the endoscope according to the first embodiment of the present invention in the state before filling the filler;
FIG. 3A is a longitudinal cross-sectional view showing an initial stage of a filler filling step in a manufacturing process of the distal end forming portion of the endoscope according to the first embodiment of the present invention;
FIG. 3B is a longitudinal cross-sectional view showing a middle stage of the filler filling step in the manufacturing process of the distal end forming portion of the endoscope according to the first embodiment of the present invention;
FIG. 3C is a longitudinal cross-sectional view showing a final stage of the filler filling step in the manufacturing process of the distal end forming portion of the endoscope according to the first embodiment of the present invention;
FIG. 4 is a schematic perspective view showing a distal end forming portion of an endoscope according to a second embodiment of the present invention in a state before filling a filler;
FIG. 5 is a schematic perspective view showing a distal end forming portion of an endoscope according to a third embodiment of the present invention in a state before filling a filler;
FIG. 6 is a schematic perspective view showing a distal end forming portion of an endoscope according to a fourth embodiment of the present invention in a state before filling a filler;
FIG. 7 is a schematic perspective view showing a distal end forming portion of an endoscope according to a fifth embodiment of the present invention in a state before filling a filler;
FIG. 8 is a schematic perspective view showing a distal end forming portion of an endoscope according to a sixth embodiment of the present invention in a state before filling a filler; and
FIG. 9 is a longitudinal cross-sectional view showing a distal end forming portion of an endoscope according to a seventh embodiment of the present invention in a state before filling a filler.

### Best Mode for Carrying out the Invention

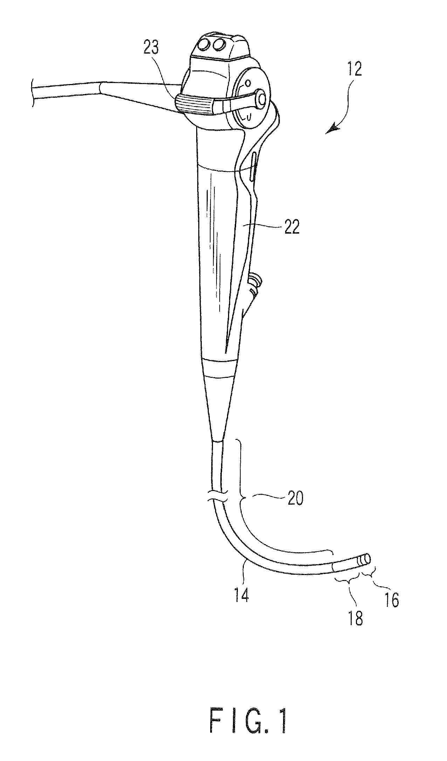

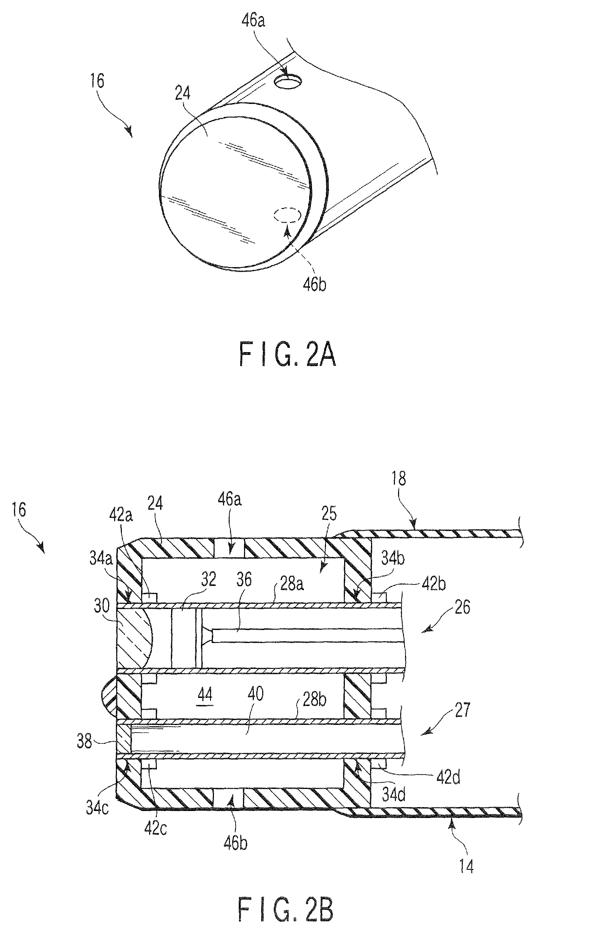

A first embodiment according to the present invention will now be explained hereinafter with reference to FIGS. 1 to 3C. As shown in FIG. 1, an endoscope 12 according to this embodiment is an electronic endoscope. This endoscope 12 includes an elongated insertion portion 14 that is inserted into a body cavity. This insertion portion 14 is formed by sequentially coupling a distal end forming portion 16, a bending portion 18 that is operated to bend, and a long flexible tube portion 20 including flexibility from a distal end side. Various kinds of built-in components such as an observation optical system 26 (see FIG. 2B), an illumination optical system 27 (see FIG. 2B), an accessory insertion channel and others are inserted through the insertion portion 14 from the distal end forming portion 16, and led to an operating portion 22 coupled with a proximal end of the insertion portion 14. A bending operation lever 23 that is used to bend the bending portion 18 is arranged at the operating portion 22 that is held and operated by an operator.

The distal end forming portion 16 before filling a filler will now be explained with reference to FIGS. 2A and 2B. The distal end forming portion 16 includes a distal end member 24 coupled with a distal end of the bending portion 18. This distal end member 24 is a substantially cylindrical hollow member including a front end wall, a rear end wall, and a circumferential wall. The distal end member 24 is formed by fitting a cover member forming the front end wall to a main body member forming the circumferential wall and the rear end wall.

The inside of the distal end member 24 forms a built-in component accommodating portion 25 that accommodates distal ends of the various kinds of built-in components therein. That is, a first frame member 28a is arranged at the distal end of the observation optical system 26, an object lens group 30 and an image device 32 are arranged in an inner hole of this first frame member 28 in the order from the distal end side and fixed to the first frame member 28a. On the other hand, a first front end insertion hole 34a and a first rear end insertion hole 34b are formed to pass through the front end wall and the rear end wall of the distal end member 24 to be aligned in a longitudinal direction of the insertion portion 14, respectively. Additionally, the first frame member 28a is fitted in the first front end insertion hole 34a, extends through the built-in component accommodating portion 25 along the longitudinal direction, and inserted and fitted in the first rear end insertion hole 34b. It is to be noted that an image signal transmission cable 36 extends from the image device 32, and this cable 36 extends from the first frame member 28a to be inserted through the insertion portion 14.

Further, a second frame member 28b is arranged at the distal end of the illumination optical system 27. An illumination lens 38 is arranged at a distal end of an inner hole of this second frame member 28b and fixed thereto. A light guide 40 for transmission of illumination light is inserted and fitted in the second frame member 28b and fixed thereto, and a distal end surface of the light guide 40 is connected with a rear end surface of the illumination lens 38. The second frame member 28b is inserted and fitted in a second front end insertion hole 34c, extends through the built-in component accommodating section 25 in the longitudinal direction, and inserted and fitted in a second rear end insertion hole 34d like the first frame member 28a. It is to be noted that the light guide 40 extends from the second frame member 28b to be inserted through the insertion portion 14.

Distal ends of the various kinds of built-in components are held by the built-in component holding portion and positioned with respect to the distal end member 24. That is, in regard to the observation optical system 26, an annular first front end holding member 42a and an annular first rear end holding member 42b are arranged on a rear end surface of the front end wall and a rear end surface of the rear end wall of the distal end member 24 so that they are substantially coaxial with the first front end insertion hole 34a and the first rear end insertion hole 34b. The first frame member 28a is inserted and fitted in these first front end holding member 42a and the first rear end holding member 42b by interference fit. The first front end holding member 42a and the first rear end holding member 42b are accurately arranged with respect to the distal end member 24, and the first frame member 28a is accurately positioned with respect to the distal end member 24. Further, like the observation optical system 26, the second frame member 28b is accurately positioned with respect to the distal end member 24 through a second front end holding member 42c and a second rear end holding member 42d.

As explained above, the first front end holding member 42a, the first rear end holding member 42b, the second front end holding member 42c, and the second rear end holding member 42d form the built-in component holding portion.

A part of the built-in component accommodating portion 25 except a part occupied by the built-in components forms a filler accommodating portion 44 that is filled with a filler. Furthermore, a first communicating portion 46a for filling and a second communicating portion 46b for air ventilation that allow the outside of the distal end member 24 to communicate with the filler accommodating portion 44 are formed to pass through a circumferential wall of the distal end member 24. The first communicating portion 46a and the second communicating portion 46b are substantially symmetrically arranged with respect to a central axis of the distal end member 24.

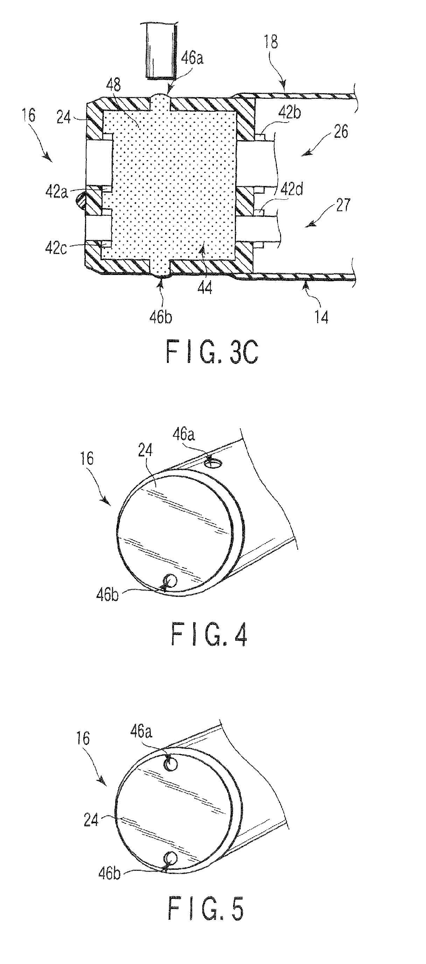

It is to be noted that structures other than the distal end member 24 and the first and the second communicating portions 46a and 46b are omitted in FIG. 2A to facilitate understanding the illustration. This can be also applied to FIGS. 4 to 8.

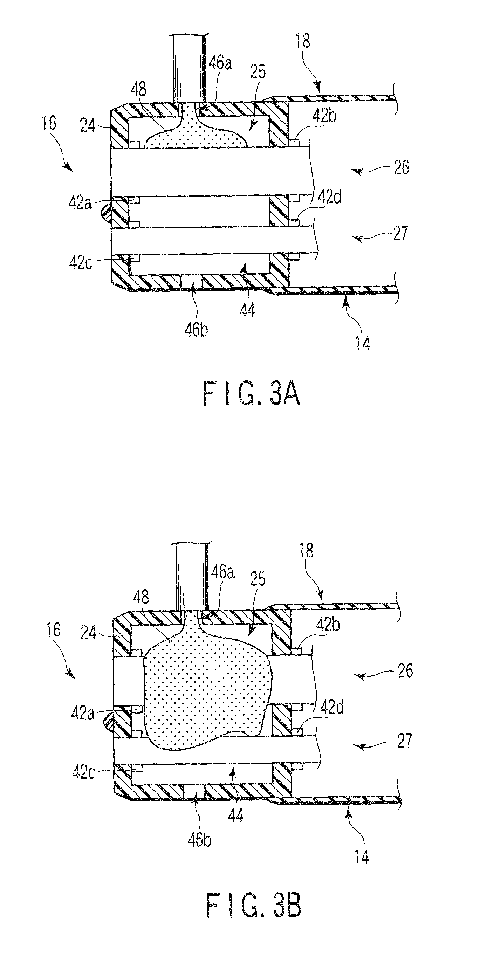

A step of filling a filler 48 in the filler accommodating portion 44 will now be explained with reference to FIGS. 3A to 3C. It is to be noted that the distal end member 24 is held at the filling step in such a manner that an outward opening direction of the second communicating portion 46b becomes opposite to a direction of a gravitational force. This is also applied to the following embodiments. As shown in FIG. 3A, the filler 48 is injected into the filler accommodating portion 44 through the first communicating portion 46a. With injection of the filler 48, air in the filler accommodating portion 44 is discharged from the second accommodating portion 46b. When the filler 48 is injected, an outer peripheral surface of each built-in component is covered with the filler 48 as shown in FIG. 3B, thereby wrapping the built-in component. As shown in FIG. 3C, injection of the filler 48 is stopped immediately after the filler 48 protrudes from the second communicating portion 46b. It is to be noted that the first and the second communicating portions 46a and 46b are also filled with the filler 48. When the filler 48 overflows through the second communicating portion 46b, the overflowing filler 48 is wiped off. When the filled filler 48 is solidified, the distal end forming portion 16 is brought to completion.

It is to be noted that the filler accommodating portion 44 does not have to be fully filled with the filler 48 when filling the filler 48 in the filler accommodating portion 44, and it is good enough for the filler 48 to cover a part of each built-in component in the built-in component accommodating portion 25, thereby enabling fixation of the built-in component. Even if the filler accommodating portion 44 is not fully filled with the filler 48, the second communicating portion 46b functions as an air vent, and a filling status can be observed through the second communicating portion 46b.

Here, the filler is appropriately selected in accordance with fixation strength, thermal insulation characteristics, water-tight characteristics and the like required for the distal end forming portion 16. For example, as the filler, a two component epoxy resin, a silicon resin, or an ultraviolet cure resin is used.

Therefore, the endoscope 12 according to this embodiment demonstrates the following effects. In this embodiment, the distal end of each built-in component is accommodated in the built-in component accommodating portion 25 in the distal end member 24, the distal end of the built-in component is positioned with respect to the distal end member 24 by the built-in component holding portion, the filler is filled in the filler accommodating portion 44 from the first communicating portion 46a to cover the built-in component, and the filler overflowing through the second communicating portion 46b is wiped off, thereby manufacturing the distal end forming portion 16. Therefore, the distal end member 24 does not have to be intricately and accurately machined, and an operation of filling the filer or an operation of wiping off the overflowing filler is easy, thus facilitating manufacture of the distal end forming portion 16. Therefore, a manufacturing cost of the endoscope 12 is reduced.

Further, a part of the built-in component accommodating portion 25 formed of the inside of the distal end member 24 except a part occupied by each built-in component forms the filler accommodating portion 44. That is, the filler accommodating portion 44 is formed of the distal end member 24, and a member required to form the filler accommodating portion 44 is not provided separately from the distal end forming portion 16, thereby simplifying the structure of the distal end forming portion 16.

FIG. 4 shows a second embodiment according to the present invention. Like reference numerals denote structures including the same functions as those in the first embodiment, thereby omitting an explanation thereof. In this embodiment, a first communicating portion 46a is formed in a circumferential wall of a distal end member 24, and a second communicating portion 46b is formed in a front end wall of the distal end member 24.

Here, like the first embodiment, if the first communicating portion 46a and the second communicating portion 46b are formed in the circumferential wall of the distal end member 24 to be symmetrical with a central axis of the distal end member 24, or if one of the first communicating portion 46a and the second communicating portion 46b is formed in a front end wall of the distal end member 24 and the other communicating portion is formed in a rear end wall of the same, one communicating portion enters a blind spot when observing the other communicating portion at a filler filling step, and simultaneously observing both the communicating portions is difficult. In this embodiment, since the first communicating portion 46a is formed in the circumferential wall of the distal end member 24 and the second communicating portion 46b is formed in the front end wall, both the communicating portions can be simultaneously observed, thus smoothly achieving the filling step.

Furthermore, when the second communicating portion 46b is formed in the circumferential wall of the distal end member 24, the filler must be wiped off on the curved circumferential surface, and the wipe-off operation is relatively troublesome. In this embodiment, since the second communicating portion 46b is formed in the front end wall of the distal end member 24, the wipe-off operation is carried out on a flat front end surface, and hence the wipe-off operation becomes relatively easy.

FIG. 5 shows a third embodiment according to the present invention. Like reference numerals denote structures including the same functions as those in the first embodiment, thereby omitting an explanation. In this embodiment, first and second communicating portions 46a and 46b are formed in a front end wall of a distal end member 24.

At a filling step, in a state where a front end surface of the distal end member 24 is held to face opposite to a direction of a gravitational force, a filler is filled from the first communicating portion 46a on the front end surface while observing the second communicating portion 46b on the front end surface to confirm a filling status, and the filler is wiped off on the front end surface when the filler overflows through the second communicating portion 46b. As explained above, in this embodiment, the filling step can be brought to completion on the front end surface alone of the distal end member 24, and the filling step can be further smoothly achieved.

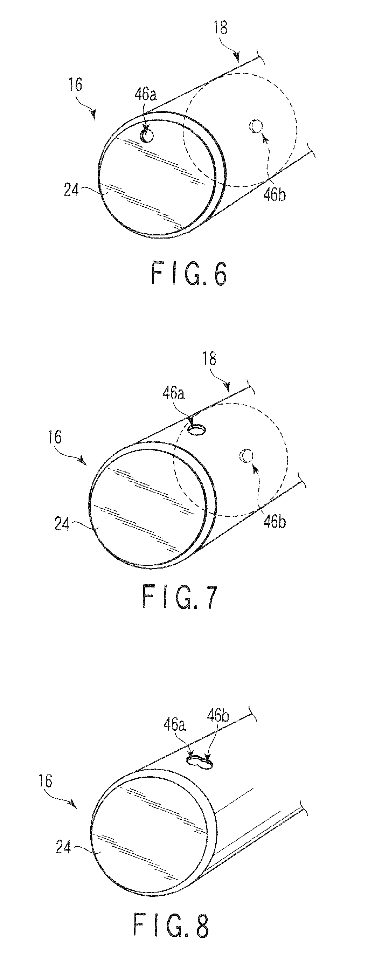

FIG. 6 shows a fourth embodiment according to the present invention. Like reference numerals denote structures including the same functions as those in the first embodiment, thereby omitting an explanation. In this embodiment, a first communicating portion 46a is formed in a front end wall of a distal end member 24, and a second communicating portion 46b is formed in a rear end wall of the distal end member 24. Further, the distal end forming portion 16 is coupled with a distal end of a bending portion 18 after a filler is filled into the distal end member 24 to form a distal end forming portion 16.

In this embodiment, since the second communicating portion 46b is formed in the rear end wall of the distal end member 24, the filler overflows on the rear end surface of the distal end member 24 that is not exposed to the outside, and the filler does not have to be carefully wiped off in terms of at least an appearance quality, thereby facilitating a wipe-off operation.

FIG. 7 shows a fifth embodiment according to the present invention. Like reference numerals denote structures including the same functions as those in the first embodiment, thereby omitting an explanation. In this embodiment, a first communicating portion 46a is formed in a circumferential wall of a distal end member 24, and a second communicating portion 46b is formed in a rear end wall of the distal end member 24. This embodiment demonstrates the same functions and effects as those in the second and the fourth embodiments.

FIG. 8 shows a sixth embodiment according to the present invention. Like reference numerals denote structures including the same functions as those in the first embodiment, thereby omitting an explanation. In this embodiment, first and second communicating portions 46a and 46b are formed in a circumferential wall of a distal end member 24 to be continuous with each other. At a filling step, a straw-like filler injection pipe is inserted into the distal end member 24 from the first communicating portion 46a, and a filler is injected into a filler accommodating portion 44 from the filler injection pipe while gradually pulling out the filler injection pipe from the distal end member 24.

In this embodiment, since the first communicating portion 46a and the second communicating portion 46b are formed in the circumferential wall of the distal end member 24 to be continuous with each other, when piercing the distal end member 24 to form the first communicating portion 46a and the second communicating portion 46 therein, slightly changing a posture of the distal end member 24 after a piercing processing of one communicating portion enables shifting to a piercing processing of the other communicating portion, thus facilitating machining of the distal end member 24.

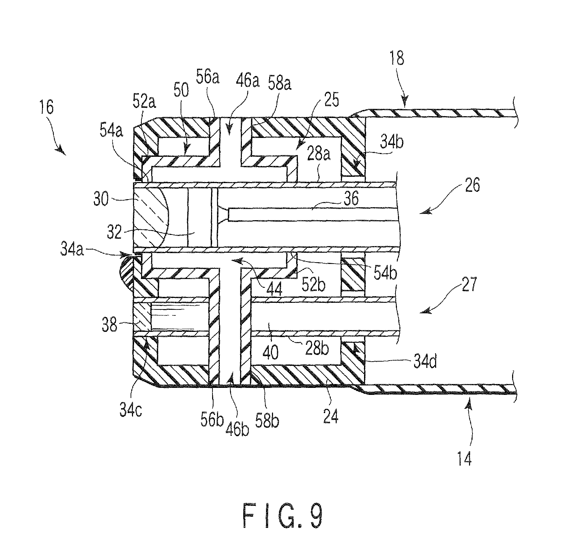

FIG. 9 shows a seventh embodiment according to the present invention. Like reference numerals denote structures including the same functions as those in the first embodiment, thereby omitting an explanation. In this embodiment, the inside of a hollow filler accommodating member 50 arranged in a distal end member 24 forms a filler accommodating portion 44.

A distal end forming portion 16 before filling a filler will now be explained with reference to FIG. 9. The substantially cylindrical hollow filler accommodating member 50 including a front end wall, a rear end wall, and a circumferential wall extends in a built-in component accommodating portion 25 in the distal end member 24 along a longitudinal direction of an insertion portion 14. A distal end of the filler accommodating member 50 is fitted and fixed in a fit concave portion formed on a rear end surface of a front end wall of the distal end member 24.

The front end wall and the rear end wall of the filler accommodating member 50 will the referred to as a front end holding wall 52a and a rear end holding wall 52b hereinafter, respectively. A front end holding hole 54a and a rear end holding hole 54b are formed to pass through the front end holding wall 52a and the rear end holding wall 52b of the filler accommodating member 50, respectively. A first frame member 28a of an observation optical system 26 is inserted and fitted in the front end holding hole 54a, extends through the filler accommodating member 50 along the longitudinal direction, and is inserted and fitted in the rear end holding hole 54b. Here, the first frame member 28a is inserted and fitted in the front end holding hole 54a and the rear end holding hole 54b by interference fit to be positioned with respect to the distal end member 24. As explained above, according to this embodiment, the front end holding wall 52a and the rear end holding wall 52b of the filler accommodating member 50 forms a built-in holding member that holds a distal end of the observation optical system 26 as a built-in component to be positioned with respect to the distal end member 24.

A part of the inside of the filler accommodating member 50 except a part occupied by the observation optical system 26 as a built-in component forms a filler accommodating portion 44 that is filled with the filler. Moreover, first and second cylindrical members 56a and 56b extend from a circumferential wall of the filler accommodating member 50. Extended ends of the first and the second cylindrical members 56a and 56b are fitted in first and second circumferential wall insertion holes 58a and 58b formed in the circumferential wall of the distal end member 24 and fixed thereto, respectively. Inner holes of the first and the second cylindrical members 56a and 56b form first and second communicating portions 46a and 46b that allow the outside of the distal end member 24 to communicate with the filler accommodating portion 44.

Further, a filler filling step is the same as that in the first embodiment.

Therefore, the endoscope 12 according to this embodiment demonstrates the following effects. In this embodiment, the inside of the hollow filler accommodating member 50 arranged in the distal end member 24 forms the filler accommodating portion 44. Therefore, the filler accommodating portion 44 can be set at an arbitrary position in the distal end member 24, and the filler can be applied to a necessary part alone of an outer peripheral surface of the built-in component, thereby wrapping the built-in component. Accordingly, the distal end forming portion 16 can be manufactured with a necessary minimum amount of the filler, and a material cost can be reduced, in particular, when using an expensive filler.

## Claims

1. An endoscope distal end forming portion **characterized by** comprising:
a hollow distal end member provided at a distal end of an elongated endoscope insertion portion that is inserted into a body cavity;
a built-in component accommodating portion that is formed of an inside of the distal end member, and accommodates a distal end of an elongated built-in component which is inserted through the insertion portion;
a built-in component holding portion that holds the distal end of the built-in component to be positioned with respect to the distal end member;
a filler accommodating portion that is provided inside of the distal end member;
a first and a second communicating portions that allow the outside of the distal end member to communicate with the filler accommodating portion; and
a filler that is accommodated in the filler accommodating portion from the first communicating portion with the second communicating portion being used as an air vent, and covers at least a part of the built-in component.

2. The endoscope distal end forming portion according to claim 1, **characterized in that**
the filler accommodating portion is formed of a part of the built-in component accommodating portion except a part occupied by the built-in component.

3. The endoscope distal end forming portion according to claim 1, **characterized in that**
the distal end member includes a substantially cylindrical shape including both end walls and a circumferential wall,
one of the first and the second communicating portions is formed to pass through one end wall, and
the other of the first and the second communicating portions is formed to pass through the circumferential wall.

4. The endoscope distal end forming portion according to claim 1, **characterized in that**
the distal end member includes a substantially cylindrical shape including both end walls and a circumferential wall, and
the second communicating portion is formed to pass through one end wall.

5. The endoscope distal end forming portion according to claim 1, **characterized in that**
the distal end member includes a substantially cylindrical shape including both end walls and a circumferential wall, and
the first and the second communicating portions are formed to pass through one end wall.

6. The endoscope distal end forming portion according to claim 1, **characterized in that**
the distal end member includes a substantially cylindrical shape including a front end wall, a rear end wall, and a circumferential wall, and
the second communicating portion is formed to pass through the rear end wall.

7. The endoscope distal end forming portion according to claim 1, **characterized in that**
the first and the second communicating portions are formed to pass through the distal end member in such a manner that the first and the second communicating portions are continuous with each other.

8. The endoscope distal end forming portion according to claim 1, **characterized in that**
the endoscope distal end forming portion further comprises a hollow filler accommodating member that is provided in the distal end member and includes the filler accommodating portion formed therein.

9. The endoscope distal end forming portion according to one of claims 1 to 8, **characterized in that**
the built-in component includes an observation optical system.

10. An endoscope **characterized by** comprising the endoscope distal end forming portion according to one of claims 1 to 9.
